# EUROPEAN PATENT APPLICATION

(11) **EP 4 621 791 A1**
(43) Date of publication of application: **24.09.2025**
(21) Application number: 24753309.4
(22) Date of filing: 05.02.2024
(51) Int. Cl.: G16H 20/00

(54) **MEDICAL CARE ASSISTANCE SYSTEM**

(30) Priority: 09.02.2023 JP 2023018371
(71) Applicant: Physiologlas Technologies, Inc., Sagamihara-shi, Kanagawa 252-0373 (JP)
(72) Inventor: MIYAWAKI Kazuyoshi, Sagamihara-shi, Kanagawa 252-0373 (JP); KOKUBO Kenichi, Sagamihara-shi, Kanagawa 252-0373 (JP); AOKI Hiroyuki, Sagamihara-shi, Kanagawa 252-0373 (JP)
(74) Representative: Schön, Christoph
(86) International application number: PCT/JP2024/003717
(87) International publication number: WO 2024/166866

(57) **Abstract**

The present invention provides a system that can support medical practitioners in providing appropriate treatment related to a patient's lifestyle for blood purification treatment of the patient. After a model's determination or learning, the measurement result of a first specified state variable p₁ immediately before and/or immediately after the blood purification treatment of the patient as a subject is acquired through an input interface 11 by a medical care assistance processing element 20. The measurement result of the first specified state variable p₁ immediately before and/or immediately after the blood purification treatment of the patient is input into a model constructed through machine learning as described above as input data by the medical care assistance processing element 20. In response, the medical care assistance processing element 20 acquires a score as output data of the model and outputs the score through an output interface 12.

## Description

### Technical Field

The present invention relates to a technique for assisting medical practitioners in treatment related to blood purification treatment.

### Background Art

In the related art, a technique for generating renal failure blood treatment prescriptions for patients has been proposed (see, for example, Patent Literature 1). Specifically, the parameters of the kinetic model specific to a patient are first identified based on the concentration of the solute to be removed (such as urea, β2-M and/or phosphate) in blood samples taken from the patient a plurality of times. The target concentration of the solute in the patient's blood is then input into this kinetic model. As a result, a treatment prescription, including duration of treatment, frequency of treatment, dialysate flow rate and/or blood flow rate to achieve the target concentration of the solute in question, is proposed. In addition, the patient's lifestyle is taken into account in the relevant treatment prescription to determine the final method of treatment.

### Citation List

### Patent Literature

Patent Literature 1: Japanese Patent No. 6018567

### Summary of Invention

### Technical Problem

In this regard, it may be desirable for both the medical practitioners and the patient to provide an appropriate lifestyle for the patient's treatment prescription.

Therefore, it is an object of the present invention to provide a system that can assist treatment by medical practitioners related to the patient's lifestyle appropriate for the blood purification treatment of the patient.

### Solution to Problem

A medical care assistance system of the present invention inputs, into a model, a measurement result of a first specified state variable indicating a patient's condition at least one of immediately before and immediately after blood purification treatment as input data, and outputs, from the model, a score indicating suitability of a patient's lifestyle during a specified period that includes a non-treatment period when the blood purification treatment was not performed in the past, as output data.

In the medical care assistance system having the above-described configuration, it is preferable that the system outputs a determination result of whether the score is above a threshold value.

In the medical care assistance system having the above-described configuration, it is preferable that, based on the score, the system outputs an estimation value of a second specified state variable indicating a matter for improving the patient's lifestyle.

### Brief Description of Drawings

FIG. 1 is a diagram illustrating a configuration of a medical care assistance system according to an embodiment of the present invention.
FIG. 2 is a functional explanatory diagram of the medical care assistance system according to the embodiment of the present invention.

### Description of Embodiments

### (Configuration)

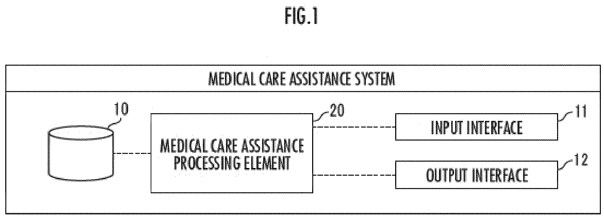

A medical care assistance system according to an embodiment of the present invention illustrated in FIG. 1 includes a database 10, an input interface 11, an output interface 12, and a medical care assistance processing element 20.

The database 10 is configured to store and hold a measurement result, a model, and the like indicating a patient' condition, which will be described later. The database 10 may be configured by a database server separate from the medical care assistance system.

The input interface 11 is configured with a keyboard, a touch panel, an imaging device, and/or a microphone (audio input device), and is configured to receive an input of information related to a patient in response to a keyboard operation, operations such as a touch, a tap, a swipe, a pinch, a gesture and/or a speech of a user such as a medical practitioner.

The output interface 12 is configured by a monitor (image display device, touch panel type display) and/or a speaker, and is configured to output information for assisting treatment of a patient by a user such as a medical practitioner.

The medical care assistance processing element 20 is configured by an arithmetic processing device (CPU, processor, and/or processor core, or the like), a storage device (memory such as ROM and RAM, HDD, SSD, and the like), an interface circuit, and the like. The medical care assistance processing element 20 is configured to execute various kinds of arithmetic processing described below by reading out necessary data and a program (software) from a storage device (which may constitute the database 10) by an arithmetic processing device and executing arithmetic processing on the data according to the program.

### (Functions)

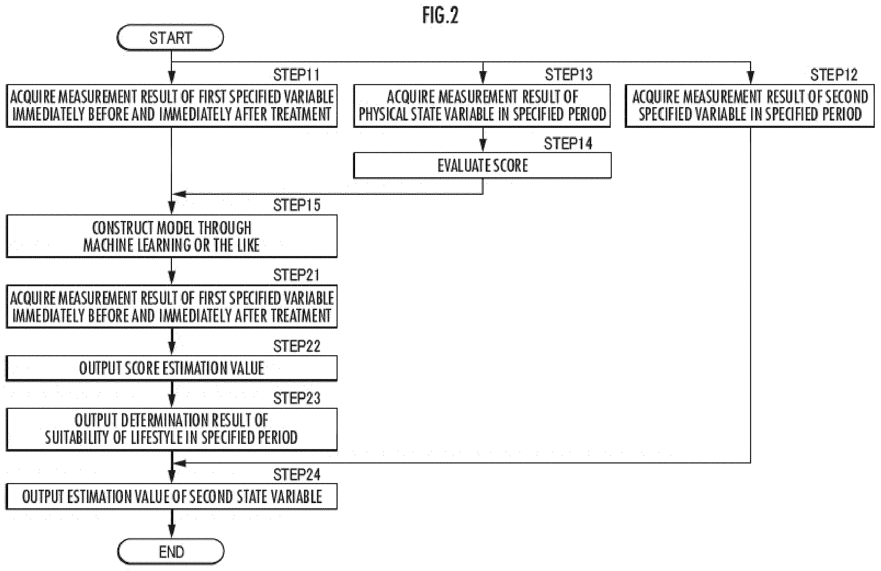

With the medical care assistance system having the above-described configuration, the measurement result of a first specified state variable p₁ immediately before and/or immediately after the current blood purification treatment of various patients is acquired through the input interface 11 by the medical care assistance processing element 20 (FIG. 2/STEP 11). The measurement result of the first specified state variable p₁ is stored and held or registered in the database 10.

The "first specified state variable p₁" includes, for example, the concentration of the first specified substance in the patient's blood, as well as blood pressure, weight, body fat percentage, BMI, estimated bone mass, muscle mass and/or total body water, and the like. The first specified substance includes, for example, uremic substances and/or uremic toxins (K, Ca, Na, phosphate), free small molecule substances (urea, creatinine, uric acid, and the like), protein-bound small molecule substances (homocysteine, and the like), medium molecule substances (leptin, and the like), albumin and/or hemoglobin.

In addition, the measurement result of a second specified state variable p₂ indicating a patient' lifestyle in a specified period including the non-treatment period before the current blood purification treatment of the patient is acquired through the input interface 11 by the medical care assistance processing element 20 (FIG. 2/STEP 12). The measurement result of the second specified state variable p₂ is stored and held or registered in the database 10.

The "second specified state variable p₂" includes the number of meals, meal start time, meal end time, meal duration, amount of water consumed, intake of various nutrients (for example, protein, carbohydrates, lipids, various vitamins, and various minerals (Ca, K, Na, and the like)), type of medicine (specific numerical value according to type), time and number of times of taking medicine, index values indicating lifestyle habits (for example, amount of alcohol intake, frequency, smoking frequency, sleep duration (time to go to bed, time to wake up), amount of exercise (number of steps)), score indicating the presence or severity of physical illness (high fever, fatigue, vomiting), and the like.

Further, the medical care assistance processing element 20 acquires the measurement result over the specified period (length T) of the physical state variable p indicating the physical state of the patient through the input interface 11 (FIG. 2/STEP 13). The measurement result of the physical state variable p is stored and held or registered in the database 10. The "physical state variable p" includes, for example, the concentration of the second specified substance in the patient's blood, blood pressure, weight, body fat percentage, muscle mass, total body water, activity level, amount of exercise, sleep duration, heart rate, respiratory rate and/or body temperature, and the like. The second specified substance may be the same as or different from the first specified substance. The second specified substance may include, for example, uremic substances and/or uremic toxins (K, Ca, Na, phosphate, free small molecule substances (urea, creatinine, uric acid, and the like), protein-bound small molecule substances (homocysteine, and the like), medium molecule substances (leptin, and the like)).

Based on the measurement results of various patients' physical state variables, a score SC is evaluated by the medical care assistance processing element 20 (FIG. 2/STEP 14). The "score SC" is an index value indicating the suitability of the patient's lifestyle over the specified period, and the score SC is evaluated to be lower as a total value ΣₜΔp(t) or an average value (ΣₜΔp(t)/T) of a deviation Δp(t) (= |p₀(t) - p(t)|) between a temporal variation pattern p(t) (a curve indicating the temporal variation pattern) of a measured value of a physical state variable p (a scalar or a vector) and a temporal variation pattern p₀(t) of a target value p₀ of the physical state variable p is larger, and/or as the frequency at which the measured value of the physical state variable p is out of a target range [p₀₋, p₀₊] is higher. For example, the score SC is determined according to a reduction function of x (ds/dx < 0) such as score SC(x) = exp(-c₁x) (0 < c₁), s(x) = 1/{1 + exp(c₁x)}, s(x) = c₂/αx², (0 < c₁, 0 < α), or an evaluation table equivalent thereto, with a total value ΣₜΔp(t) or the like of the deviations as the variable x (0 ≤ x).

Then, the medical care assistance processing element 20 uses the measurement result (learning input data) of a first specified state variable p₁ and the evaluation result (learning output data or label) of the score SC as learning data or training data, and constructs a model (score estimation model) through machine learning (FIG. 2/STEP 15). As a machine learning method, a known training or learning method such as linear regression analysis, quantification theory, support vector machine (SVM), decision tree, random forest, k-nearest neighbor method, Naive Bayes (simple Bayes classifier), and neural network, or a method equivalent thereto can be employed. A plurality of models may be constructed according to differences in the attributes of the patients. The attributes of the patient are classified by age, gender, and/or physical features (such as height and weight, at least a part of the first specified state variable p₁) or the like.

After the model is determined or trained, the measurement result of the first specified state variable p₁ immediately before and/or immediately after the blood purification treatment of a target patient is acquired through the input interface 11 by the medical care assistance processing element 20 (FIG. 2/STEP 21). The target patient may be the same as or different from the patient from which the learning data is acquired in a case of constructing the model. The measurement result of the first specified state variable p₁ immediately before and/or immediately after the blood purification treatment of the patient is input to the model (or the model corresponding to the attributes of the target patient) constructed through the machine learning as described above as input data for estimation by the medical care assistance processing element 20. In response, an estimation value SCₑₛₜ of the score SC is acquired as output data (output data for estimation) of the model by the medical care assistance processing element 20, and is output to the output interface 12 (FIG. 2/STEP 22).

The determination result of the suitability of the patient's lifestyle in a specified period is displayed on the output interface 12 according to the score estimation value SCₑₛₜ of the patient by the medical care assistance processing element 20 (FIG. 2/STEP 23). In a case where the score estimation value SCₑₛₜ of the patient is equal to or greater than a threshold value SCₜₕ, it is determined that the patient's lifestyle in the specified period is suitable. On the contrary, in a case where the score estimation value SCₑₛₜ of the patient is less than the threshold value SCₜₕ, it is determined that the patient's lifestyle in the specified period is not suitable. In addition, the determination result of the degree or the rank of the suitability of the patient's lifestyle in the specified period may be displayed on the output interface 12, depending on which of a plurality of numerical ranges the score estimation value SCₑₛₜ falls into.

The medical care assistance processing element 20 selects another patient having a score SC (score constituting training data) having a specified relationship with the score estimation value SCₑₛₜ of the patient, and the estimation value of the second specified state variable p₂ for improving the patient's lifestyle is derived based on the measurement result of the second specified state variable p₂ indicating the lifestyle, and then output to the output interface 12 (FIG. 2/STEP 24).

In a case where the score estimation value SCₑₛₜ in a current specified period of the patient and the evaluation value of the score SC in a previous specified period or earlier of the patient are in the specified relationship, the patient (the same patient) of which the score SC in the previous specified period is evaluated may be selected as the other patient. In this case, the second specified state variable p₂ (intake amount of various nutrients and the like) indicating the patient's lifestyle in the current specified period is output to the output interface 12, which represents the patient's lifestyle in the previous specified period.

For example, the specified relationship may include that the score estimation value SCₑₛₜ(i) of a patient P(i) is higher than a reference value ε (0 < ε). In this case, the measurement result of the second specified state variable p₂ of the other patient P(j) having the score SC(j) satisfying a relational expression SCₑₛₜ(i) + ε₋ ≤ SC(j) or the average value thereof is derived as the estimation value of the second specified state variable p₂ for improving the lifestyle of the patient P(i). As a result, the second specified state variable p₂ (intake amount of various nutrients and the like) indicating the lifestyle of another patient P(j) (including the same patient in the past) as an index for improving the lifestyle of the patient P(i) in a certain specified period is output to the output interface 12.

Different "specified relationships" may be defined according to the difference in the relationship between the score estimation value SCₑₛₜ(i) of the patient P(i) and the threshold value SCₜₕ.

For example, in a case where the score estimation value SCₑₛₜ(i) of the patient P(i) is equal to or greater than the threshold value SCₜₕ, the specified relationship may include that the deviation from the score estimation value SCₑₛₜ(i) is close enough to be included in a first specified range [-ε₋, ε₊] (0 ≤ ε₋, 0 < ε₊). In this case, the measurement result of the second specified state variable p₂ of the other patient P(j) having the score SC(j) satisfying a relational expression SCₑₛₜ(i) - ε₋ ≤ SC(j) ≤ SCₑₛₜ(i) + ε₊ or the average value thereof is derived as the estimation value of the second specified state variable p₂ for improving the lifestyle of the patient P(i).

In a case where the score estimation value SCₑₛₜ(i) of the patient P(i) is less than the threshold value SCₜₕ, the specified relationship may include that the deviation from the score estimation value SCₑₛₜ(i) is within a first specified range [ε₁, ε₂] (0 < ε₁< ε₂). In this case, the measurement result of the second specified state variable p₂ of the other patient P(j) having the score SC(j) satisfying a relational expression SCₑₛₜ(i) + ε₁ ≤ SC(j) ≤ SCₑₛₜ(i) + ε₂ or the average value thereof is derived as the estimation value of the second specified state variable p₂ for improving the lifestyle of the patient P(i).

### (Effect)

With the medical care assistance system according to the present invention, various types of information are output to the output interface 12, and thus, the treatment by the medical practitioner related to the patient's lifestyle, which is suitable for the blood purification treatment of the patient, can be supported.

### (Other Embodiments of Present Invention)

A model in which the measurement result of the first specified state variable p₁ is input as input data and a score is output as output data may be employed as a kinetic model (N-pool kinetic model (N = 1, 2, ...)) that simply represents the metabolism and dynamics of substances in the body. For example, a single-pool model is a model that represents dynamics in which urea is removed from a living body considered as a single container and at the same time urea is generated in the container. The two-pool model is a model that represents the dynamics in which urea is removed from a living body, which is considered to consist of two compartments, an intracellular compartment and an extracellular compartment, and at the same time urea is generated in the container. In addition to the amount of dialysis such as Kt/V and/or a urea removal rate calculated by these models, an increase function and/or a reduction function with nPCR or at least one of Kt/V and urea removal rate as a variable may be used as a score (output data). The model parameters that define the kinetic model may be predetermined or may be identified by machine learning.

The input data is input to each of a plurality of models selected from one or a plurality of models constructed through machine learning and one or a plurality of kinetic models, and the total value, the average value, or the weighted average value of the scores as the output data from each model is evaluated as a total score, and the determination processing of the suitability of the patient's lifestyle in the specified period or the like may be executed based on the total score (see FIG. 2/STEP 21 to 24).

In the embodiment, the measurement result of the first specified state variable p₁ is included in the input data, but in another embodiment, in addition to the measurement result of the first specified state variable p₁ of the patient, an environment variable (average temperature, maximum temperature, minimum temperature, humidity, weather, rainfall, or the like) indicating an environment in which the patient is exposed during the specified period may be included.

The second specified state variable p₂ (learning input data) and the physical state variable p (learning output data) may be input to the input interface 11, and the model (model for estimating physical state variables) may be constructed or generated by the medical care assistance processing element 20 through machine learning. In this case, the physical state variable p of the subject may be estimated by the medical care assistance processing element 20 by inputting the second specified state variable p₂ (input data for estimation) of the subject whose physical state variable p is unknown to the model through the input interface 11. The estimation result or the score SC corresponding to the estimation result may be output to the output interface 12. Each of the learning input data and the input data for estimation may include the first specified state variable p₁ in addition to the second specified state variable p₂.

The physical state variable p (learning input data) and the second specified state variable p₂ (learning output data) may be input to the input interface 11, and a model (model for estimating physical state variables) may be constructed or generated by the medical care assistance processing element 20 through machine learning. In this case, the second specified state variable p₂ of the subject may be estimated by the medical care assistance processing element 20 by inputting the physical state variable p (input data for estimation) of the subject whose the second specified state variable p₂ is unknown, to the model through the input interface 11. The estimation result may be output to the output interface 12. Each of the learning input data and the input data for estimation may include the first specified state variable p₁ in addition to the physical state variable p.

### Description of Reference Numerals

10: database
11: input interface
12: output interface
20: medical care assistance processing element

## Claims

1. A medical care assistance system that inputs, into a model, a measurement result of a first specified state variable indicating a patient's condition of at least one of immediately before and immediately after blood purification treatment as input data, and outputs, from the model, a score indicating suitability of patient's lifestyle during a specified period that includes a non-treatment period when the blood purification treatment was not performed in the past, as output data.

2. The medical care assistance system according to Claim 1,
wherein the system outputs a determination result of whether the score is equal to or more than a threshold value.

3. The medical care assistance system according to Claim 1,
wherein based on the score, the system outputs an estimation value of a second specified state variable indicating a matter for improving the patient's lifestyle.
